Europäisches Patentamt

European Patent Office

Office européen des brevets

(11) Numéro de publication: **0 455 529 B1**

# FASCICULE DE BREVET EUROPEEN

(45) Date de publication de fascicule du brevet: **22.03.95**

(51) Int. Cl.6: **C07C 55/07**, C07C 51/41, C01F 17/00

(21) Numéro de dépôt: **91400979.0**

(22) Date de dépôt: **12.04.91**

Le dossier contient des informations techniques présentées postérieurement au dépôt de la demande et ne figurant pas dans le présent fascicule.

(54) **Procédé de fabrication d'oxalates doubles de terres rares et d'ammonium et leurs utilisations pour la fabrication d'oxydes de terres rares, oxalates doubles et oxydes obtenus.**

(30) Priorité: **04.05.90 FR 9005654**

(43) Date de publication de la demande: **06.11.91 Bulletin 91/45**

(45) Mention de la délivrance du brevet: **22.03.95 Bulletin 95/12**

(84) Etats contractants désignés: **AT BE CH DE DK ES FR GB GR IT LI LU NL SE**

(56) Documents cités:
DE-A- 2 145 820
GB-A- 1 208 565
GB-A- 2 205 090
US-A- 4 891 163

PATENT ABSTRACTS OF JAPAN, vol. 4, no. 61 (C-9)[543], 8 mai 1980;& JP-A-55 28 905 (MITSUBISHI KASEI KOGYO) 29-.2-1980

J.Inorg. Nucl., vol. 26, 1964, M. Barret et al "Double ammonium oxalates of the rare earths and Yttrium", pages 931-6

(73) Titulaire: **RHONE-POULENC CHIMIE**
**25, Ouai Paul Doumer**
**F-92408 Courbevoie Cédex (FR)**

(72) Inventeur: **Dissaux, Bernard-Antoine**
**1 rue des Sureaux**
**F-17139 Chagnolet par Dompierre sur Mer (FR)**
Inventeur: **Le Loarer, Jean-Luc**
**24 avenue du Général Guillaumat**
**F-17000 La Rochelle (FR)**
Inventeur: **Pacaud, Bernard**
**Shippo-Dai 4602-14**
**Niihma, Ehime Ken (JP)**
Inventeur: **Ries, Michel**
**5 Allée des Noyers**
**F-94400 Vitry sur Seine (FR)**

(74) Mandataire: **Esson, Jean-Pierre et al**
**RHONE-POULENC CHIMIE,**
**Direction de la Propriété Industrielle,**
**25, quai Paul Doumer**
**F-92408 Courbevoie Cédex (FR)**

Acta Cryst.,vol. 23, 1967, T. McDonald et al "The Crystal structure of a double Oxalate of Yttrium and ammonium", pages 944-9

Bull. Chem. Soc. Japan, vol. 63, Fev.1990, Y. Minagawa et al, "New preparative method of fine powder of Yttrium oxide", pages 378-82

Bull. Chem. Soc. Japan, vol 63, Jul.1990, Y. Minagawa et al, "Preparative method of fine powder of Yttrium oxide", pages 2115-7

## Description

La présente invention concerne un procédé de fabrication d'oxalates doubles de terres rares et d'ammonium, leur utilisation pour l'obtention d'oxyde de terres rares et les produits obtenus.

Elle se rapporte plus particulièrement à un procédé permettant d'obtenir des oxalates doubles présentant une morphologie et granulométrie déterminées.

Les oxydes de terres rares trouvent de nombreuses applications dans le domaine notamment de la céramique et de l'électronique mais à l'heure actuelle, on constate sur le marché une demande croissante de produits à granulométrie contrôlée.

Une des voies classiques pour obtenir des oxydes de terres rares et qui est largement décrite dans la littérature, notamment dans le NOUVEAU TRAITE DE CHIMIE MINERALE, Tome VII, (1959), P. 1007 de Paul PASCAL, consiste à calciner entre 500 et 900°C les oxalates de terres rares obtenus par précipitation à l'aide de l'acide oxalique, des sels de terres rares sous forme de solution aqueuse. Cependant, un tel procédé de fabrication ne conduit qu'à des oxydes de terres rares présentant une grosse granulométrie.

Il est également connu selon JP 53 095911-A (Chemical Abstracts 90, 40940 w) de préparer des oxydes de terres rares fins et plus particulièrement de l'oxyde d'yttrium fin par calcination d'un oxalate d'yttrium et d'ammonium qui consiste à partir d'une solution aqueuse d'un sel d'yttrium, à précipiter l'yttrium sous la forme de son hydroxyde préparé par réaction de la solution aqueuse d'un sel d'yttrium et d'une solution aqueuse basique comme l'ammoniaque puis à traiter la bouillie d'hydroxyde résultante par l'acide oxalique, et enfin à séparer le précipité obtenu, à le laver et à le calciner à une température de 750°C. Ledit procédé conduit conformément à la description donnée dans JP 53-095911-A à l'obtention d'oxyde d'yttrium fin. Le diamètre des particules est compris entre 0,9 et 4,5 $\mu$m, les cristaux ayant une forme de plaquettes à bords arrondis.

Le document Bull. Chem. Soc. Japan, vol 63, pages 378-82 (1990) décrit un mode de préparation classique d'oxalate double dans lequel une solution aqueuse d'ammoniaque est introduit dans une solution d'oxalate d'yttrium.

Toutefois, la dimension des particules est encore relativement élevée par rapport aux dimensions requises pour certaines applications comme la luminescence. En outre, le contrôle de la dimension des particules est relativement difficile car les conditions de mise en oeuvre du procédé influent fortement sur celle-ci.

Un des buts de la présente invention est notamment de remédier à ces inconvénients en proposant un procédé de fabrication d'oxalates doubles d'ammonium et de terres rares présentant une distribution granulométrique resserrée, avec des dimensions moyennes des cristaux pouvant être inférieures au micron.

Par oxalate double de terres rares et d'ammonium, il faut comprendre un composé comprenant une ou plusieurs terres rares permettant après calcination de produire des oxydes simples ou mixtes.

A cet effet, l'invention propose un procédé de fabrication d'un oxalate double de terres rares et d'ammonium caractérisé en ce qu'il consiste :

- à ajouter dans une première solution contenant des ions oxalate et d'ammonium, une seconde solution contenant un composé de terres rares
- à séparer le précipité obtenu
- et, éventuellement le sécher.

Selon une caractéristique de l'invention, le composé de terres rares est avantageusement un composé soluble comme un sel de terres rares.

Parmi les sels convenables pour l'invention, on peut citer à titre d'exemple les chlorures, les nitrates, les sulfates ou acétates de scandium, d'yttrium, de lanthane, de cérium, de praséodyme, de néodyme, de samarium, d'europium, de gadolinium, de terbium, de dysprosium, d'hofnium, d'erbium, de thulium, d'ytterbium, de lutécium ou un mélange de ceux-ci. En particulier, on peut mettre en oeuvre une solution aqueuse contenant des sels de terres rares qui proviendrait directement ou indirectement du traitement des minerais de terres rares.

Bien que le procédé de l'invention s'applique tout-à-fait bien aux terres rares cériques, il convient plus particulièrement aux terres rares yttriques.

On entend par "terres rares cériques", les éléments les plus légers des terres rares commençant avec le lanthane et s'étendant jusqu'au néodyme conformément au numéro atomique et l'on désigne par "terres rares yttriques" les éléments les plus lourds des terres rares conformément au numéro atomique, commençant avec le samarium et finissant avec le lutécium et comprenant l'yttrium.

La concentration en composé de terres rares n'est pas critique.

Cette seconde solution est une solution aqueuse dont le pH peut être ajusté à une valeur déterminée ou dans une zone déterminée par addition de solution tampon ou d'acides, si cela est nécessaire.

Généralement cette seconde solution contient essentiellement un ou plusieurs composés de terres rares.

Comme acides, on peut citer les acides minéraux tels que l'acide nitrique.

La première solution contient des ions ammonium et oxalate apportés sous forme d'un ou plusieurs sels contenant ces deux espèces tel que, par exemple, l'oxalate d'ammonium ou l'oxalate acide d'ammonium, ou par un mélange de composés apportant chacun une des deux espèces ammonium ou oxalate. Ainsi, on peut citer à titre d'exemple, les différents sels d'ammonium comme le nitrate, le chlorure d'ammonium ou même l'ammoniac sous forme de gaz ou en solution pour l'addition de l'ion ammonium.

Quant à l'espèce oxalate, on peut citer les différents oxalates métalliques comme l'oxalate de sodium par exemple, et de manière préférée l'acide oxalique.

La concentration en ions $(C_2O_4)^=$ et $NH_4^+$ dans la solution n'est pas critique et peut varier dans de larges limites.

Selon un mode de réalisation préféré, on déterminera les concentrations en oxalate et ammonium ainsi que la concentration en terre rare dans la seconde solution et les volumes des première et seconde solution pour obtenir en fin d'addition de la seconde solution un rapport molaire entre l'ion oxalate et les terres rares $((C_2O_4)^=/TR)$ supérieur ou égal à 2, avantageusement supérieur à 2,5 et un rapport ammonium à terres rares $(NH_4^+/TR)$ supérieur ou égal à 2, de préférence supérieur à 2,5.

Dans un mode de réalisation de l'invention, la première solution est une solution d'oxalate d'ammonium.

Selon un autre mode de réalisation de l'invention, la première solution est une solution d'oxalate d'ammonium et d'acide oxalique. Avantageusement, le rapport molaire $(C_2O_4)^=/NH_4^+$ est supérieur à 2 et compris entre 2 et 4.

Selon un autre mode de réalisation de l'invention, la première solution est une solution d'oxalate d'ammonium et d'ammoniac, le rapport $C_2O_4^=/NH_4^+$ doit être supérieur à 0, et est alors avantageusement compris entre 0,4 et 2.

Cette première solution peut également comprendre un acide minéral (HA) tel que l'acide nitrique, par exemple. Cet acide pouvant être ajouté à une solution d'oxalate d'ammonium ou libéré in situ par addition d'acide oxalique à une solution de sels d'ammonium, par exemple de nitrate d'ammonium.

Quand un acide minéral HA est présent dans la première ou seconde solution, la quantité d'acide HA engagée est déterminée pour obtenir un rapport molaire de l'anion A- dudit acide par rapport à la quantité totale d'oxalate inférieur à 5.

Les conditions de mise en oeuvre du procédé sont peu critiques pour obtenir un oxalate double. Toutefois, le contrôle de la vitesse d'introduction de la seconde solution, de la température, de l'agitation du mélange permet de modifier et contrôler la morphologie de l'oxalate double précipité.

Il est également possible de mettre en oeuvre le procédé de l'invention de manière continue par mélange en continu des première et seconde solutions décrites ci-dessous.

Par ailleurs, la température a une influence sur le rendement de la précipitation car le coefficient de solubilité de l'oxalate double augmente avec l'élévation de la température.

Le précipité obtenu est séparé du liquide surnageant par tout procédé de séparation solide/liquide comme par exemple, filtration, centrifugation décantation ou analogue. Il peut également être soumis à un ou plusieurs lavages, pour, par exemple, éliminer les sels solubles.

L'oxalate double de terres rares et d'ammonium peut subir un séchage pour évaporer l'eau non lié par exemple par un traitement thermique entre 50°C et 100°C ou un séchage sous pression réduite.

L'oxalate double de terres rares et d'ammonium obtenu par le procédé de l'invention est constitué de particules dont la forme et les dimensions dépendent de la nature et du pH des solutions ainsi que des conditions de température, d'agitation et vitesse d'introduction pendant le mélange des solutions.

Ainsi, dans le cas où la première solution est une solution d'oxalate d'ammonium, le produit obtenu est constitué de particules sous forme de plaquettes de faible épaisseur (épaisseur égale à environ 0,3 $\mu$m) et de dimension moyenne comprise entre 0,5 $\mu$m et 3 $\mu$m, avantageusement entre 0,5 $\mu$m et 1 $\mu$m.

Dans le cas où la première solution est une solution d'un mélange d'acide oxalique et d'ammoniaque ou d'un autre composé d'ammonium à l'exception de l'oxalate d'ammonium, la dimension moyenne des particules varie entre 1 $\mu$m et 5 $\mu$m. Ces particules sont de forme sensiblement cubique.

Le procédé de l'invention permet de produire un oxalate double de terres rares et d'ammonium présentant une granulométrie homogène. Ainsi, la répartition granulométrique des cristaux est très resserrée. Le coefficient de dispersion des tailles des cristaux est généralement inférieur à 0,7, avantageusement compris entre 0,2 et 0,6.

Par coefficient de dispersion on entend le rapport

$$\frac{\emptyset_{84} - \emptyset_{16}}{2\emptyset_{50}}$$

dans laquelle $\emptyset_{84}$ $\emptyset_{16}$ $\emptyset_{50}$ représentent les diamètres des particules correspondant à 84 %, 16 % et 50 % de celles-ci.

Une des utilisations de ces oxalates doubles de terres rares et d'ammonium est la production d'oxyde de terres rares obtenues par décomposition thermique de ceux-ci.

La morphologie et granulométrie des oxydes de terres rares obtenus par décomposition d'un oxalate double est généralement semblable à celle dudit oxalate double utilisé comme précurseur. Toutefois, selon les conditions de traitement thermique de l'oxalate double, la granulométrie de l'oxyde peut être légèrement différente de celle de l'oxalate.

Le traitement thermique ou calcination est généralement réalisée à une température comprise entre 600 et 1200 °C, avantageusement entre 800 °C et 1000 °C.

La durée de calcination est déterminée de manière classique par le contrôle du poids constant. A titre indicatif, la durée de la calcination peut varier entre 30 minutes et 6 heures environ.

L'invention sera illustrée par des exemples donnés ci-dessous uniquement à titre indicatif.

SERIE I D'EXEMPLES

On ajoute une solution de nitrate d'yttrium à 170 g/l exprimé en $Y_2O_3$ dans une solution d'oxalate d'ammonium de concentration variable inférieure à 0,9 M.

Les différents essais réalisés sont rassemblés dans le tableau 1 ci-dessous.

La taille et la répartition granulométrique des produits obtenus sont déterminées à l'aide de l'instrument SEDIGRAPH 5000D qui détermine le taux de sédimentation des particules en suspension et détermine la distribution granulométrique en pourcentages cumulatifs en fonction des diamètres équivalents sphériques. Cette détermination est basée sur la loi de STOKE.

| Rapport $NH_4/Y$ | $[Y]$ $gY_2O_3/l$ ( 1) | $D_{50}$ $\mu m$ | Coefficient de dispersion granulométrique |
|---|---|---|---|
| 5 | 5 | 0,69 | 0,57 |
| 5 | 17 | 0,84 | 0,47 |
| 6 | 17 | 0,80 | 0,35 |
| 8 | 17 | 0,64 | 0,53 |

avec

$[Y]$ : concentration totale en yttrium dans le milieu réactionnel exprimée en $Y_2O_3$

$D_{50}$ : dimension moyenne de l'oxyde de terre rare obtenu par calcination de l'oxalate double (900 °C pendant 1 heure).

Les particules ont une forme de plaquette à contours assez irrégulier.

SERIE II D'EXEMPLES

Cette série d'exemples est réalisée avec une seconde solution de nitrate d'yttrium identique à la première série, mais avec une première solution contenant un mélange de $(NH_4)_2C_2O_4$ et $H_2C_2O_4$ ;

Les résultats et rapports entre les différentes espèces mises en oeuvre sont rassemblés dans le tableau II. La concentration en Y dans le milieu réactionnel est de 17 g/l en $Y_2O_3$.

| $C_2O_4^=/Y$ | $NH_4^+/Y$ | $D_{50}$ oxyde | Coefficient de répartition granulométrique (F) |
|---|---|---|---|
| 2,5 | 2,5 | 0,90 | 0,46 |
| 3,0 | 3,0 | 1,15 | 0,67 |
| 4,0 | 5,0 | 1,1 | 0,40 |

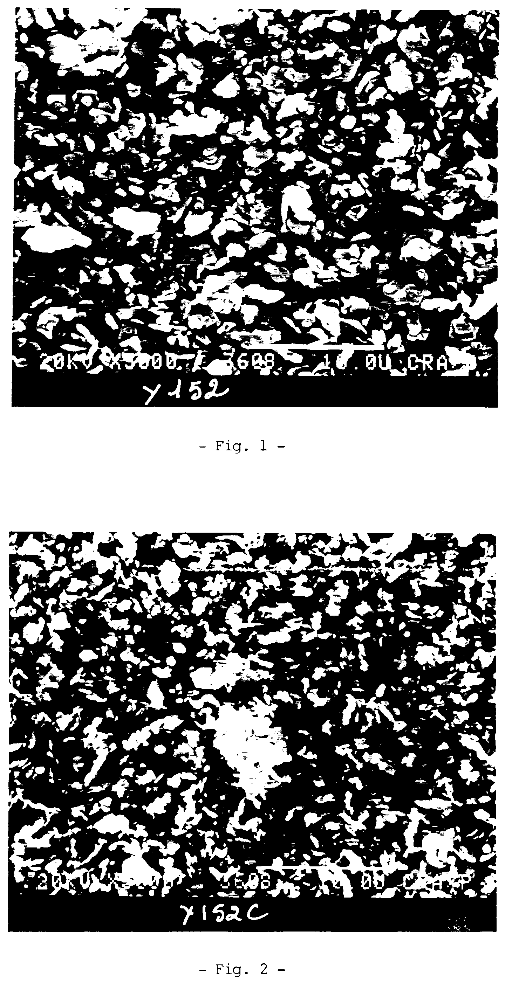

Les cristaux d'oxalate double obtenus sont sous forme de plaquette d'épaisseur 0,3 $\mu$m environ et de dimensions moyennes 0,7 et 1 $\mu$m. Les morphologies de l'oxalate et de l'oxyde calciné sont illustrées respectivement dans les figures 1 et 2.

SERIE III D'EXEMPLES

Exemples identiques à ceux de la série II, mais avec une première solution qui comprend de l'ammoniaque et de l'acide oxalique.

Les résultats et rapports entre les différentes espèces sont donnés dans le tableau III (concentration finale en Yttrium : 17 g/l en $Y_2O_3$)

| $C_2O_4^=/Y$ | $NH_4^+/Y$ | $D_{50}$ oxyde | Coefficient de dispersion granulométrique (F) |
|---|---|---|---|
| 4,0 | 3,0 | 1,5 | 0,37 |
| 2,5 | 5,0 | 0,75 | 0,47 |

EXEMPLE IV

On ajoute une solution de nitrate d'yttrium contenant 150 g/l exprimé en $Y_2O_3$ et 0,8 mole/l d'acide nitrique dans une solution d'oxalate d'ammonium à 25 g/l. La quantité de solution engagée est déterminée pour obtenir un rapport entre l'oxalate d'ammonium et le sel de terre rare supérieur de 10 % au rapport stoechiométrique.

Après séparation, séchage et calcination de l'oxalate d'ammonium et d'yttrium, on obtient un oxyde d'yttrium de $D_{50}$ égal à 2 $\mu$m avec un indice de dispersion granulométrique (F) de 0,5.

EXEMPLE V

On reproduit l'exemple IV. Toutefois, la solution de nitrate d'yttrium ne contient pas d'acide nitrique, mais au contraire, la solution d'oxalate d'ammonium contient 0,2 mole/l de $HNO_3$.

L'oxyde d'yttrium obtenu après calcination de l'oxalate d'ammonium et d'yttrium présente un $D_{50}$ de 2,3 $\mu$m avec un indice de dispersion granulométrique égal à 0,45.

SERIE VI D'EXEMPLES

L'exemple 1 est reproduit mais en utilisant une solution de sels solubles de terres rares à 170 g/l en $TR_2O_3$ dont 148 g/l ($Y_2O_3$) de nitrate d'yttrium et 22 g/l ($La_2O_3$) de nitrate de lanthane.

La solution de précipitation contient 25 g/l d'oxalate d'ammonium.

Les quantités de solutions engagées sont déterminées pour avoir un rapport molaire $NH_4/TR$ égale à 4.

Le précipité obtenu est un oxalate double d'yttrium de lanthane et d'ammonium.

Après calcination à 900°C, on obtient un oxyde mixte de lanthane et d'yttrium contenant 12,6 % de $La_2O_3$ et dont les caractéristiques sont indiquées dans le tableau ci-dessous.

| Rapport $NH_4/Y$ | [TR] g/l $(TR_2O_3)$ | $D_{50}$ µm | Coefficient de dispersion dispersion granulométrique (F) |
|---|---|---|---|
| 4 | 10 | 0,85 | 0,68 |
| 4 | 40 | 0,9 | 0,70 |

EXEMPLE VII

On prépare un oxalate double d'ammonium par mélange en continu d'une solution de nitrate d'yttrium à 170 g/l (exprimé en $Y_2O_3$) avec une solution d'oxalate d'ammonium à 25 g/l.

Ainsi, le rapport $NH_4/Y$ est de 4,75 et la concentration finale en $Y_2O_3$ est de 16 g/l.

Le précipité récupéré après séparation et lavage est un oxalate double d'yttrium et d'ammonium présentant un diamètre moyen des particules égal à 1,4 µm avec un coefficient de dispersion granulométrique (F) égal à 0,4.

**Revendications**

**1.** Procédé de fabrication d'un oxalate double de terres rares et d'ammonium caractérisé en ce qu'il consiste :
- à ajouter dans une première solution contenant des ions oxalate et d'ammonium, une seconde solution contenant un composé de terres rares
- à séparer le précipité obtenu
- et, éventuellement le sécher.

**2.** Procédé selon la revendication 1 caractérisé en ce que la première solution est une solution d'oxalate d'ammonium.

**3.** Procédé selon la revendication 1 caractérisé en ce que la première solution est une solution d'acide oxalique et d'un composé d'ammonium.

**4.** Procédé selon la revendication 3, caractérisé en ce que le composé d'ammonium est choisi dans le groupe comprenant l'ammoniaque, l'oxalate d'ammonium, un sel d'ammonium et d'acide minéral ou un mélange de ceux-ci.

**5.** Procédé selon la revendication 4 caractérisé en ce que le sel d'ammonium est un nitrate ou un chlorure d'ammonium.

**6.** Procédé selon les revendications 2 à 5, caractérisé en ce que la première solution contient un acide minéral (HA).

**7.** Procédé selon l'une des revendications précédentes, caractérisé en ce que la seconde solution contient un sel soluble de terres rares choisi dans le groupe des nitrates, chlorures, sulfates ou un mélange de sels de terres rares.

**8.** Procédé selon l'une des revendications 1 à 7, caractérisé en ce que la seconde solution comprend un acide minéral (HA)

9. Procédé selon la revendication 7 ou 8 caractérisé en ce que la seconde solution est une solution de nitrate d'yttrium, nitrate d'europium, nitrate de lanthane, nitrate de néodyme, nitrate de dysprosium, cérium, gadolinium, terbium ou un mélange de ceux-ci.

10. Procédé selon l'une des revendications précédentes, caractérisé en ce que le rapport molaire $C_2O_4^=/TR$ et $NH_4^+/TR$ à la fin de la précipitation est supérieur à 2, de préférence supérieur à 2,5.

11. Procédé selon l'une des revendications précédentes, caractérisé en ce que dans la première solution, le rapport molaire $(C_2O_4)^=/NH_4^+$ est inférieur à 4 et supérieur à 0.

12. Procédé selon la revendication 6 ou 8, caractérisé en ce que la quantité d'acide minéral (HA) dans la première solution ou la seconde solution est déterminée pour avoir un rapport entre l'anion A- et les ions oxalates inférieur à 5 dans le milieu réactionnel après mélange.

13. Procédé selon l'une des revendications 1 à 12, caractérisé en ce qu'il consiste à mélanger en continu la première et la seconde solution.

14. Procédé de fabrication d'oxyde de terres rares, caractérisé en ce qu'il consiste à calciner l'oxalate double d'ammonium et de terre rare obtenu selon l'une des revendications précédentes, après un séchage éventuel.

15. Procédé selon la revendication 14, caractérisé en ce que la température de calcination est comprise entre 600 et 1200°C, de préférence entre 800°C et 1000°C.

16. Oxalate double d'ammonium et de terre rare, caractérisé en ce qu'il est cristallisé sous forme de plaquette de dimension moyenne comprise entre $0,5\mu$m et $3\mu$m avec un coefficient de dispersion granulométrique inférieur à 0,7.

17. Oxyde de terres rares, caractérisé en ce qu'il est constitué par des particules en forme de plaquette de dimension moyenne comprise entre $0,5\mu$m et $3\mu$m avec un coefficient de dispersion granulométrique inférieur à 0,7.

## Claims

1. A process for the production of a rare earth and ammonium double oxalate, characterised in that it consists in:
   - adding a second solution containing a rare earth compound to a first solution containing oxalate and ammonium ions;
   - separating the precipitate obtained; and
   - drying if required.

2. Process according to claim 1, characterised in that the first solution is an ammonium oxalate solution.

3. Process according to claim 1, characterised in that the first solution is a solution of oxalic acid and an ammonium compound.

4. Process according to claim 3, characterised in that the ammonium compound is selected from the group comprising ammonia, ammonium oxalate, an ammonium salt of a mineral acid, or a mixture thereof.

5. Process according to claim 4, characterised in that the ammonium salt is an ammonium nitrate or chloride.

6. Process according to claims 2 to 5, characterised in that the first solution contains a mineral acid (HA).

7. Process according to any one of the preceding claims, characterised in that the second solution contains a soluble rare earth salt selected from the group formed by nitrates, chlorides and sulphates or a mixture of rare earth salts.

8. Process according to any one of claims 1 to 7, characterised in that the second solution comprises a mineral acid (HA).

9. Process according to claim 7 or claim 8, characterised in that the second solution is a solution of yttrium nitrate, europium nitrate, lanthanum nitrate, neodymium nitrate, or dysprosium, cerium, gadolinium or terbium nitrate, or a mixture thereof.

10. Process according to any one of the preceding claims, characterised in that the $C_2O_4^=$/RE and $NH_4^+$/RE molar ratio following precipitation is greater than 2, preferably greater than 2.5.

11. Process according to any one of the preceding claims, characterised in that the $(C_2O_4)^=$/$NH_4^+$ molar ratio in the first solution is less than 4 and greater than 0.

12. Process according to claim 6 or claim 8, characterised in that the quantity of mineral acid (HA) in the first solution or the second solution is determined so that the ratio between the anion $A^-$ and the oxalate ions in the reaction mixture following mixing is less than 5.

13. Process according to any one of claims 1 to 12, characterised in that it consists in mixing the first and second solutions in a continuous process.

14. A process for production of rare earth oxides, characterised in that it consists in calcining the double ammonium and rare earth oxalate obtained in accordance with any one of the preceding claims, following drying if required.

15. Process according to claim 14, characterised in that the calcining temperature is between 600°C and 1200°C, preferably between 800°C and 1000°C.

16. A double ammonium and rare earth oxalate, characterised in that it is crystallized in the form of platelets with an average dimension of between 0.5 $\mu$m and 3 $\mu$m and a granulometric dispersion coefficient of less than 0.7.

17. A rare earth oxide, characterised in that it is constituted by particles in platelet form with an average dimension of between 0.5 $\mu$m and 3 $\mu$m with a granulometric dispersion coefficient of less than 0.7.

**Patentansprüche**

1. Verfahren zur Herstellung eines Doppeloxalates der seltenen Erden und von Ammonium, dadurch gekennzeichnet, daß es besteht in:
   - dem Zusammenfügen einer ersten Lösung, die Oxalat- und Ammoniumionen enthält, und einer zweiten Lösung, die eine Zusammensetzung der seltenen Erden enthält,
   - dem Abtrennen des erhaltenen Niederschlages,
   - und gegebenenfalls dem Trocknen.

2. Verfahren nach Anspruch 1, dadurch gekennzeichnet, daß die erste Lösung eine Ammoniumoxalat-Lösung ist.

3. Verfahren nach Anspruch 1, dadurch gekennzeichnet, daß die erste Lösung eine Lösung von Oxalsäure und einer Ammoniumverbindung ist.

4. Verfahren nach Anspruch 3, dadurch gekennzeichnet, daß die Ammoniumverbindung aus der Gruppe gewählt wird, die umfaßt: Ammoniak, Ammoniumoxalat, ein Ammoniumsalz einer Mineralsäure oder deren Mischung.

5. Verfahren nach Anspruch 4, dadurch gekennzeichnet, daß das Ammoniumsalz ein Nitrat oder ein Chlorid von Ammonium ist.

6. Verfahren nach Anspruch 2 bis 5, dadurch gekennzeichnet, daß die erste Lösung eine Mineralsäure (HA) enthält.

9

**7.** Verfahren nach einem der vorstehenden Ansprüche, dadurch gekennzeichnet, daß die zweite Lösung ein lösliches Salz der seltenen Erden enthält, gewählt aus der Gruppe der Nitrate, Chloride, Sulfate oder einer Mischung dieser Salze der seltenen Erden.

**8.** Verfahren nach einem der Ansprüche 1 bis 7, dadurch gekennzeichnet, daß die zweite Lösung eine Mineralsäure (HA) umfaßt.

**9.** Verfahren nach Anspruch 7 oder 8, dadurch gekennzeichnet, daß die zweite Lösung eine Lösung von Yttriumnitrat, Europiumnitrat, Lanthannitrat, Neodymnitrat, Dysprosiumnitrat, Cernitrat, Gadoliniumnitrat, Terbiumnitrat oder deren Mischung ist.

**10.** Verfahren nach einem der vorstehenden Ansprüche, dadurch gekennzeichnet, daß das molare Verhältnis $(C_2O_4)^=$/TR und $NH_4^+$/TR am Ende der Fällung höher ist als 2, vorzugsweise höher als 2,5.

**11.** Verfahren nach einem der vorstehenden Ansprüche, dadurch gekennzeichnet, daß in der ersten Lösung das molare Verhältnis $(C_2O_4)^=$/$NH_4^+$ unterhalb von 4 und höher als 0 beträgt.

**12.** Verfahren nach Anspruch 6 oder 8, dadurch gekennzeichnet, daß die Menge von Mineralsäure (HA) in der ersten Lösung oder in der zweiten Lösung bestimmt wird, um in dem Reaktionsmedium nach dem Mischen ein Verhältnis zwischen dem Anion A- und den Oxalationen unterhalb von 5 vorliegen zu haben.

**13.** Verfahren nach einem der Ansprüche 1 bis 12, dadurch gekennzeichnet, daß es im kontinuierlichen Mischen der ersten und der zweiten Lösung besteht.

**14.** Verfahren zur Herstellung von Oxid der seltenen Erden, dadurch gekennzeichnet, daß es darin besteht, das nach einem der vorstehenden Ansprüche erhaltene Doppeloxalat von Ammonium und seltener Erde, gegebenenfalls nach einer Trocknung, zu kalzinieren.

**15.** Verfahren nach Anspruch 14, dadurch gekennzeichnet, daß die Temperatur der Kalzinierung zwischen 600 °C und 1200 °C, vorzugsweise zwischen 800 °C und 1000 °C liegt.

**16.** Doppeloxalat von Ammonium und seltener Erde, dadurch gekennzeichnet, daß es in Form von Plättchen der mittleren Dimension zwischen 0,5 $\mu$m und 3 $\mu$m mit einem granulometrischen Dispersions-Koeffizienten von unter 0,7 kristallisiert ist.

**17.** Oxid der seltenen Erden, dadurch gekennzeichnet, daß es aus Teilchen in Form von Plättchen der mittleren Dimension zwischen 0,5 $\mu$m und 3 $\mu$m mit einem granulometrischen Dispersions-Koeffizienten von unter 0,7 besteht.

- Fig. 1 -

- Fig. 2 -